Europäisches Patentamt

European Patent Office

Office européen des brevets

(11) Publication number: 0 371 748
A2

# (12) EUROPEAN PATENT APPLICATION

(21) Application number: 89312329.9

(22) Date of filing: 28.11.89

(51) Int. Cl.5: C07C 57/04, C07C 51/44,
C07C 51/50, C07C 69/54,
C07C 67/54, C07C 67/62

(30) Priority: 29.11.88 US 277097

(43) Date of publication of application:
06.06.90 Bulletin 90/23

(84) Designated Contracting States:
AT BE CH DE ES FR GB GR IT LI LU NL SE

(71) Applicant: HOECHST CELANESE
CORPORATION
Route 202-206 North
Somerville, N.J. 08876(US)

(72) Inventor: Levy, Leon B.
6130 Coralridge
Corpus Christi Texas(US)
Inventor: Scates, Mark O.
1801 Crooked Creek
Pearland Texas(US)

(74) Representative: De Minvielle-Devaux, Ian
Benedict Peter et al
CARPMAELS & RANSFORD 43, Bloomsbury
Square
London WC1A 2RA(GB)

(54) Inhibition of polymerization during distillation of monomers.

(57) Unintentional or premature polymerization of ethylenically unsaturated organic compounds, such as acrylic acid and monomeric acrylates, during distillation is inhibited by means of a phenolic inhibitor and a manganese or cerium alkanoate of a $C_5$ to $C_{19}$ monocarboxylic acid. Compositions of ethylenically unsaturated organic compounds with phenolic inhibitors and manganese or cerium alkanoates are also described.

EP 0 371 748 A2

# INHIBITION OF POLYMERIZATION DURING DISTILLATION OF MONOMERS

## BACKGROUND OF THE INVENTION

### 1. Field of the Invention

The present invention relates to a method for inhibiting unintentional or premature polymerization of ethylenically unsaturated organic compounds, such as acrylic acid and monomeric acrylates, during the distillation thereof, and more particularly to the distillation of ethylenically unsaturated organic compounds in the presence of certain liquid phase inhibitors. The invention also relates to compositions of ethylenically unsaturated organic compounds and certain liquid phase inhibitors.

### 2. Description of the Prior Art

Ethylenically unsaturated organic compounds, such as acrylic acid, methacrylic acid, methacrylic esters, acrylate esters, and the like, are widely used in the production of homopolymers and copolymers. These homopolymers and copolymers, produced readily through the polymerization of the available double bonds of the organic compounds, are widely used in paints, coatings, lacquers and the like. The olefinic activity of the ethylenically unsaturated organic compounds, makes the polymerized products highly useful for many purposes. On the other hand, the olefinic activity poses a problem of unintentional or premature polymerization promoted by light and heat. This tendency is exhibited on storage, shipping, and during purification of these polymerizable unsaturated compounds where distillation at higher temperatures are involved. During distillation the liquid phase in the distillation unit should contain a liquid phase non-volatile inhibitor and the column and condenser may also contain a volatile inhibitor such as nitric oxide to prevent the undesired or premature polymerization of the ethylenically unsaturated organic monomer product.

U. S. 2,741,583 to Vaughn et al discloses the use of alkali metal nitrites, such as sodium nitrite, or a mixture of nitrogen oxides as obtained by the action of an acid on such a nitrite, as polymerization inhibitors during the distillation of methyl or ethyl acrylates. British Patent GB 1,265,419 discloses a method for minimizing polymerization of acrylic acid during distillation thereof by distilling the acrylic acid in the presence of nitric oxide in the gas phase and phenothiazine in the liquid phase.

It is also known to use a metal additive in combination with phenolic inhibitors to prevent undesirable polymerization of ethylenically unsaturated organic compounds. From Chemical Abstracts, Vol. 74, 1971, 112642V, a Japanese patent (No. 70-35,285) filed June 1, 1967, assigned to Nippon Kayaku Co., Ltd., describes the mixture of chromium acetate with hydroquinone as a satisfactory inhibitor for acrylic and methacrylic acid. In Japanese patent publication No. 51-98211, filed February 19, 1975, assigned to Sumitomo Chemical Co., Ltd., the combination of manganese salt and phenolic type inhibitors provides satisfactory stabilization of acrylic acid. U. S. Patents 4,507,495 and 4,542,231 also disclose the use of cerium and manganese compounds together with phenolic type inhibitors for stabilization of ethylenically unsaturated organic compounds, such as ethyl acrylate, against unintentional polymerization.

In the preparation of acrylic acid, manganese acetate, which is available commercially, is known to impart significant polymerization inhibition, especially as a co-inhibitor with phenolic inhibitors. Manganese acetate, however, suffers the disadvantage of low solubility in ethyl acrylate and acrylic acid/ethyl acrylate mixtures, and in acrylic acid solutions in other lyophillic solvents. Because of the solubility problem and since ethyl acrylate and acrylic acid-ethyl acrylate mixtures may be found in the crude product distillation columns and other fractionation columns, there is thus a limit to where the inhibitor can be added during ethyl acrylate manufacture. Other commercially available manganese salts, such as manganese naphthenate, are soluble in acrylic acid processing streams but are salts of complicated mixtures of acids dissolved in mineral oil which act as contaminants.

## SUMMARY OF THE INVENTION

The present invention is directed to an improvement in the distillation of readily polymerizable

ethylenically unsaturated organic compounds such as acrylic acid and ethyl acrylate, by subjecting such compounds to distallation in the presence of a liquid phase inhibitor system comprising a phenolic type inhibitor and a soluble manganese and/or cerium alkanoate of saturated $C_5$ to $C_{19}$ monocarboxylic acids. The invention is further directed to compositions comprising unsaturated organic compounds, such as described above, with phenolic inhibitors and a soluble manganese or cerium alkanoate of saturated $C_5$ to $C_{19}$ monocarboxylic acids. The invention may also be carried out by the above procedure and to include the presence of a vapor phase inhibitor.

## DESCRIPTION OF THE INVENTION

In a preferred aspect of the invention, inhibition of polymerization may be applied to the distallation of acrylic acid esters obtained by alcohol esterification of acrylic acid, acrylic acid obtained by the catalytic oxidation of propylene, or the distallation of acrylic acid and ethyl acrylate as produced from the interaction of acrylic acid with ethylene in the presence of a sulfuric acid catalyst. As examples of the latter process, see U. S. Patent No. 3,703,539, issued November 21, 1972, to DiLiddo; U. S. Patent No. 3,539,621, issued November 10, 1970, to Cipollone et al; U. S. Patent No. 3,894,076, issued July 8, 1975, to Van Duyne et al; and U. S. Patent No. 4,490,553, issued December 25, 1984, to Chase et al. In these processes, the reaction is believed to involve the formation of intermediate sulfates from the reaction of ethylene with sulfuric acid in a reactor tower wherein the sulfates further react with acrylic acid to form ethyl acrylate. To provide a product mixture in good overall yields with high carbon efficiencies, the reaction mixture is sent from the reaction tower to a distallation train where the mixture is distilled to obtain liquid ethyl acrylate with unreacted ethylene, acrylic acid and sulfuric acid being recycled to the reactor. During distillation of the acrylate monomer, polymer formation and fouling occur due to the polymerization of uninhibited condensing vapor. Although it would be expected that polymer fouling would be inhibited by commonly used liquid phase inhibitors such as phenothiazine, hydroquinone, p-methoxyphenol and the like, the distillation of acrylate monomers is exacerbated by the polymerization of uninhibited condensing vapor (vapor phase polymerization) which does not contain a liquid phase inhibitor.

Reaction products withdrawn from the reactor tower are sent to a purification train comprising a recovery distillation tower, light ends distillation tower and finishing tower, all of which are of conventional design. In the recovery distillation tower, products withdrawn from the reactor tower are introduced through a pressure reduction valve and maintained under vacuum by conventional means so that for ethyl acrylate the pressure is typically less than about 500 mm of mercury absolute. The still pot temperature is maintained within the range of about $50°C$ to $170°C$, preferably $100°C$ to $130°C$, and the still head temperature within the range of about $28°C$ to $45°C$, preferably about $30°C$ to $40°C$. The recovery column is maintained at reduced pressures, less than atmospheric, so that the pressure is within the range of 300 to 450 mm of mercury absolute. The distillation section of the recovery distillation tower is of conventional design and may contain packing, sieve type trays or dual flow trays. The distillation section will contain an equivalent of at least four theoretical trays. The residence time of the reaction products in the base of the distillation tower should be as low as possible because at temperatures required in the reboiler for vaporization some polymerization may occur. It is desirable to have a feed stream lean in acrylic acid being fed to the recovery distillation tower since this will result in less polymer formation.

Light ends of crude ethyl acrylate, comprising mainly ethyl acrylate, small amounts of unreacted ethylene and other uncondensables, are removed overhead from the recovery distillation tower and passed to a light ends distillation tower of conventional distillation design. A stream comprising mainly unreacted ethylene is removed as overhead from the lights ends distillation tower and may be disposed of or recycled to the reactor tower as desired. The bottoms product from the light ends distillation tower is a partially purified ethyl acrylate product which is passed to a finishing distillation tower where a substantially pure ethyl acrylate product is recovered by fractionation. The ethyl acrylate residue product from the finishing distillation tower is removed as a bottoms product and can be recycled to the reactor tower or a portion thereof sent to a wiped-film evaporator for recovery of organic products such as ethyl acrylate and acrylic acid.

The manganese and/or cerium compounds used as liquid phase inhibitors in accordance with the invention are the manganese and cerium alkanoates of saturated $C_5$ to $C_{19}$ monocarboxylic acids and isomers thereof. Representative compounds include managanese pentanoate, manganese hexanoate, cerium hexanoate, manganese heptanoate, cerium heptanoate, manganese octanoate, cerium octanoate, manganese nonanoate (manganese pelargonate), cerium pelargonate, manganese decanoate, manganese undecanoate, manganese dodecanoate, manganese tridecanoate, cerium tridecanoate, manganese tetradecanoate, manganese pentadecanoate, cerium pentadecanoate, manganese hexadecanoate, manganese heptadecanoate, manganese octadecanoate, cerium octadecanoate, manganese nonadecanoate. Other useful liquid phase inhibitors include manganese and cerium naphthenates.

Preferred alkanoates are the manganese and cerium salts of $C_5$ to $C_{12}$ monocarboxylic acids such as heptanoic acid and pelargonic acid.

The above described alkanoates are sufficiently soluble at high concentrations in acrylic acid, acrylate esters and acrylates of polyols, and are effective inhibitor synergists when used with a phenolic inhibitor such as hydroquinone, hydroquinone monomethyl ether (MeHQ), etc.

It has been further found that the manganese compounds used in accordance with the invention are effective as co-inhibitors with phenolic inhibitors when the manganese exists in either the +2 and/or +3 valence state. A manganese salt such as manganese pelargonate, for example, is conveniently prepared by dissolving manganese acetate in pelargonic acid at a temperature of about 40°C to 45°C with stirring. The acetate dissolves readily to give a light pinkish solution, but over a 10 minute period there is a gradual change to a darker color which indicates an oxidation of manganese from the +2 to the more soluble +3 valence.

In Table A below, ethyl acrylate and glacial acrylic acid monomers were tested for polymerization induction periods in the presence of manganese pelargonate with the manganese existing in the +2 and/or +3 valence states. The phenolic inhibitor employed was 15 parts per million of methyl ether of hydroquinone (MEHQ) in ethyl acrylate, and 200 parts per million in acrylic acid. The testing was carried out at 100°C under a nitrogen atmosphere after having sparged the monomer with nitrogen. In each test the monomers contained 3.3 parts per million of manganese. As shown in Table A manganese in the +2 and/or +3 valence states are both synergistic with phenolic inhibitors.

TABLE A

| POLYMERIZATION INDUCTION PERIODS OF ETHYL ACRYLATE AND GLACIAL ACRYLIC ACID WITH MANGANESE(II) AND MANGANESE(III) PELARGONATES | | |
|---|---|---|
| Manganese Valence (a) | Addition Temperature, °C | Induction Period, Hr. |
| Blank | -- | 30, 37, 32, 37, 46, 49 |
| III | 25 (b) | 48 - 64 (c) |
| III | 25 | 171 - 188 |
| III | 100 | 70 - 97 |
| III | 100 | 149 - 163 |
| II (d) | 100 | 170 - 184 |
| II (d) | 100 | 196 - 212 |
| III (e) | 25 | >200 |

a. Manganese valence III indicates that the manganese acetate solution in pelargonic acid (1.57 wt.%) was heated to 100°C with air bubbling for about 15 minutes, at which time it was dark red-brown.

b. Temperature of the manganese pelargonate solution when it was added to the monomer. The monomer itself was at room temperature in all cases.

c. The monomer polymerized within the time limits shown above. The monomer was observed not to have polymerized at the first time noted, and to have visibly increased in viscosity or developed solids by the second time noted.

d. Managanese valence II indicates that the manganese acetate solution in pelargonic acid (1.57 wt.%) was treated at 100°C with nitrogen until it was colorless.

e. Glacial acrylic acid.

The amount of manganese or cerium alkanoate (expressed as the metal) employed will range from about 1 part per million to about 5,000 parts per million, preferably about 1 to 500 parts per million and most preferably from about 1 to 30 parts per million based on the reaction mixture or individual product.

In the production of ethyl acrylate, the manganese or cerium alkanoates and phenolic type polymerization inhibitors are usually added subsequent to reaction of the ethylene, acrylic and sulfuric acid. When the manganese or cerium compounds are dissolved in ethyl acrylate and synergize with the phenolic inhibitors, such as hydroquinone or the monomethyl ether thereof, the thermal stability of ethyl acrylate is increased dramatically, both in the presence and absence of oxygen. This is totally unexpected since it was not known whether manganese or cerium alkanoates would synergize with phenolic inhibitors when dissolved in a non-polar solvent and existed in ion-pair form rather than as free manganese or cerium ions, as is the case when manganese or cerium acetate is dissolved in acrylic acid. The combination of phenolic inhibitors with manganese or cerium alkanoates in ethylenically unsaturated compounds, such as described above, provide useful compositions which can be readily distilled without significant polymerization during distillation.

Vapor phase inhibitors, if desired, also may be employed in accordance with the invention and include nitric oxide (NO) or N-phenyl-N-nitrosohydroxylamine ammonium salt (NPH), or the like. In the processing of acrylic acid, ethyl acrylate and other polymerizable ethylenically unsaturated organic compounds, the combined presence of liquid and vapor phase inhibitors permits the use of increased temperatures and pressures, thereby increasing the capacity of distillation columns for purification and recovery. The amount of vapor phase inhibitor employed will range from 5 parts per million to 5,000 parts per million but in most cases, for economic reasons, the inhibitor will be used in amounts ranging from 20 to 1,000 parts per million based on the product or product mixture.

The phenolic inhibitors which are used herein include known inhibitors of the phenolic type such as dihydroxybenzene derivatives such as hydroquinone, catechol, resorcinol, dihydroxyxylene, methoxyphenols such as guaiacol and p-methoxyphenol (methyl ether of hydroquinone), pyrogallol, methylpyrogallol, cresols, phenol, xylenols, 4,4'-thiobis-(5-methyl-2-tertiary-butyl-phenol) and the like. The amounts of phenol type inhibitors which are employed will range from about 5 parts per million to about 5,000 parts per million, preferably from about 25 parts per million to about 500 parts per million based on the product or total reaction product mixture.

A wide variety of ethylenically unsaturated organic compounds may be inhibited and stabilized against unintentional or premature polymerization. Such compounds include acrylic acid, methacrylic acid, vinyl acetate, styrene, acrylonitrile, vinyl chloride, acrylamide, N-methylolacrylamide, glycidyl methacrylate, and the like; an alkyl acrylate ester wherein the alkyl group contains from 1 to 10 carbon atoms such as methyl acrylate, ethyl acrylate, n-butyl acrylate, octyl acrylate, isoctyl acrylate, 2-ethylhexyl acrylate and the like; an alkyl methacrylate ester wherein the alkyl group contains from 1 to 10 carbon atoms such as methyl methacrylate, ethyl methylacrylate, butyl methacrylate, and the like; an acrylate ester prepared by the reaction of acrylic acid and a saturated aliphatic polyol having 2 to 10 carbon atoms and 2 to 5 hydroxy groups, said ester containing 2 to 5 acrylate groups such as pentaerythritol triacrylate, trimethyl propane triacrylate, 1,6-hexandiol diacrylate, tetraethylene glycol diacrylate, tripropylene glycol diacrylate and trimethylol propane trimethacrylate and the like.

This invention will be further described in the following experiments:


EXAMPLE 1


Glacial acrylic acid and ethyl acrylate monomers were tested for polymerization induction periods in the presence of manganese pelargonate. The manganese pelargonate was added to the monomer in an amount sufficient to provide 3.5 ppm of manganese, which also introduced 0.1 wt% of pelargonic acid to the monomer. After sparging 6 grams of the monomer for 30 minutes with nitrogen or air, the liquid monomers were then sealed in a test tube with a Swagelok fitting and heated to 100°C.

In Table 1 below the phenolic inhibitor was the monomethyl ether of hydroquinone (MEHQ) which was present in the monomers as received from the supplier; 15 ppm in ethyl acrylate and 200 ppm in acrylic acid. Manganese pelargonate (nonanoate) is soluble in acrylic acid and ethyl acrylate and synergizes with the phenolic inhibitors present in the monomers. The use of manganese pelargonate, as demonstrated, imparts a dramatic increase in the thermal stability of the monomers, both in the presence and absence of air.

TABLE 1

| POLYMERIZATION INDUCTION PERIODS OF ACRYLIC ACID AND ETHYL ACRYLATE WITH MANGANESE PELARGONATE | | |
|---|---|---|
| Monomer | Atmosphere | Induction Period, hr. (b) |
| Acrylic Acid (a) | Nitrogen | 50 - 145 |
| Acrylic acid control (c) | Nitrogen | ca. 0.5 |
| Acrylic Acid (d) | Air | >9.6 |
| Acrylic Acid (d) | Air | >9.6 |
| Acrylic Acid (d) | Air | >9.6 |
| Acrylic Acid control (c) | Air | 5 - 14.5 |
| Acrylic Acid control (c) | Air | 5 - 14.5 |
| Acrylic Acid control (c) | Air | 5 - 14.5 |
| Ethyl acrylate (a) | Nitrogen | 50 - 145 |
| Ethyl acrylate control (c) | Nitrogen | 6 - 7 |
| Ethyl acrylate (a) | Air | 31 - 118 |
| Ethyl acrylate control (c) | Air | 7 - 16 |

a. Containing 3.5 ppm manganese (added as manganese pelargonate).
b. Where a range of times is indicated, the monomer was observed not to have polymerized at the first time noted, and to have visibly increased in viscosity or developed solids by the second time noted.
c. Containing no manganese pelargonate.
d. Containing 3.7 ppm manganese (added as manganese pelargonate.

EXAMPLE 2

In the following experiments, ethyl acrylate inhibited with 15 ppm hydroquinone monomethyl ether (MEHQ) was tested for thermal stability with and without manganese heptanoate in the presence and absence of oxygen. A manganese heptanoate stock solution was prepared by dissolving 0.1574 grams of manganese acetate in 9.8425 grams of heptanoic acid with stirring at room temperature. 0.0289 grams (0.035 cc) of this mixture was then added to 30.0 grams of ethyl acrylate and sparged with nitrogen for 60 minutes. 6 grams of this mixture was added to each of three test tubes which had been sparged with nitrogen for 30 minutes and sealed with a Swagelok fitting. These samples are identified as samples 1, 2 and 3. Controls were prepared by sparging 30.0 grams of ethyl acrylate with nitrogen for 60 minutes and adding 6 grams to each of three test tubes which had been sparged with nitrogen and sealed with Swagelok fittings. These samples are identified as controls 4, 5 and 6. In a similar manner, samples 7, 8 and 9 and controls 10, 11 and 12 were also prepared for testing in the presence of air. Testing for thermal stability was carried out by heating the samples and controls at 100° C in an oil bath. The results are shown in Table 2.

TABLE 2

| POLYMERIZATION INDUCTION PERIODS OF ETHYL ACRYLATE WITH MANGANESE HEPTANOATE | | |
|---|---|---|
| Ethyl Acrylate Sample | Atmosphere | Induction Period, Hr. (b) |
| Samples (a) 1 | Nitrogen | 74.25 - 138.25 |
| 2 | Nitrogen | 74.25 - 138.25 |
| 3 | Nitrogen | 74.25 - 138.25 |
| Controls (c) 4 | Nitrogen | 22.8 - 24.8 |
| 5 | Nitrogen | 28.3 - 44.5 |
| 6 | Nitrogen | 22.8 - 24.8 |
| Samples (d) 7 | Air | 49.5 - 113.5 |
| 8 | Air | 49.5 - 113.5 |
| 9 | Air | 49.5 - 113.5 |
| Controls (c) 10 | Air | 17.8 - 22.0 |
| 11 | Air | 25.0 - 41.5 |
| 12 | Air | 22.0 - 25.0 |

a. Containing 3.5 ppm manganese (added as manganese heptanoate).
b. Where a range of times is indicated, the monomer was observed not to have polymerized at the first time noted, and to have visibly increased in viscosity or developed solids by the second time noted.
c. Containing no manganese heptanoate.
d. Containing 3.3 ppm manganese (added as manganese heptanoate).

## EXAMPLE 3

Samples of acrylic acid and mixtures thereof with ethyl acrylate were prepared and tested at 100°C in the manner set forth in Example 2. Excellent thermal stability was obtained as shown below in Table 3.

TABLE 3

| POLYMERIZATION INDUCTION PERIODS OF ACRYLIC ACID AND MIXTURES WITH ETHYL ACRYLATE WITH MANGANESE HEPTANOATE | | |
|---|---|---|
| Monomer | Atmosphere | Induction Period |
| Acrylic acid (a) | | |
| Controls 1 | $N_2$ (b) | 20 min. (c) |
| 2 | $N_2$ | 20 min. |
| 3 | $N_2$ | 20 min. |
| Samples (d) 4 | $N_2$ | 75 hrs. |
| 5 | $N_2$ | >96 hrs. |
| 6 | $N_2$ | >96 hrs. |
| 50/50 Ethyl Acrylate/Acrylic Acid (a') | | |
| Controls 7 | $N_2$ | 2.2 - 18.4 hrs |
| 8 | $N_2$ | 2.2 - 18.4 hrs |
| 9 | $N_2$ | 2.2 - 18.4 hrs |
| Samples (d) 10 | $N_2$ | >72 hrs. |
| 11 | $N_2$ | >72 hrs. |
| 12 | $N_2$ | >72 hrs. |

a. Glacial acrylic acid inhibited with 200 ppm MEHQ.
a'. Inhibited with 100 ppm MEHQ (hydroquinone monomethyl ether).
b. The liquid (6 g) was sparged for 30 minutes with nitrogen and then sealed in a test tube with a Swagelok fitting.
c. The monomer polymerized within the time limits shown above. The monomer was observed not to have polymerized at the first time noted, and to have visibly increased in viscosity or developed solids by the second time noted.
d. Containing 4.5 ppm manganese (added as manganese heptanoate).

EXAMPLE 4

Samples of ethyl acrylate were prepared and tested at 100°C in the manner set forth in Example 2, using cerous acetate in place of manganese acetate to prepare a stock solution of cerous heptanoate. The samples were made up to contain 5 ppm cerium and the controls to contain no cerium. The results are shown in Table 4.

TABLE 4

| POLYMERIZATION INDUCTION PERIODS OF ETHYL ACRYLATE WITH CERIUM HEPTANOATE | | |
|---|---|---|
| Ethyl Acrylate Sample | Atmosphere | Induction Period, Hr. (b) |
| Samples (a) 1 | Nitrogen | 26.0 |
| 2 | Nitrogen | 28.5 |
| 3 | Nitrogen | 28.5 |
| Controls (c) 4 | Nitrogen | 3.5 (e) |
| 5 | Nitrogen | 8.5 - 19.0 |
| 6 | Nitrogen | 8.5 - 19.0 |
| Samples (d) 7 | Air | 5.0 - 17.5 (e) |
| 8 | Air | 27.0 - 41.5 |
| 9 | Air | 27.0 - 41.5 |
| Controls (c) 10 | Air | 17.5 - 24.5 |
| 11 | Air | 17.5 - 24.5 |
| 12 | Air | 17.5 - 24.5 |

a. Containing 5 ppm cerium (added as cerous heptanoate).
b. Where a range of times is indicated, the monomer was observed not to have polymerized at the first time noted, and to have visibly increased in viscosity or developed solids by the second time noted.
c. Containing no cerium heptanoate.
d. Containing 5 ppm cerium (added as cerous heptanoate).
e. Short induction period presumably due to contamination of sample.

## Claims

1. A method of distilling a product or product mixture comprising an ethylenically unsaturated organic compound in the presence of a phenolic inhibitor and a soluble manganese or cerium compound co-inhibitor whereby premature polymerization is inhibited, characterized in that there is used as the co-inhibitor a soluble manganese or cerium alkanoate of a saturated $C_5$ to $C_{19}$ monocarboxylic acid.

2. The method of claim 1 wherein the cerium or manganese alkanoate (expressed as ppm of the manganese or cerium metal) is present in an amount ranging from 2 parts per million to 5,000 parts per million.

3. The method of claim 1 or 2 wherein the phenolic inhibitor is selected from hydroquinone, the monomethyl ether of hydroquinone, catechol, guaiacol, and resorcinol.

4. The method of any of claims 1-3 wherein the phenolic inhibitor is added to the product or product mixture in an amount ranging from 5 parts per million to 5,000 parts per million.

5. The method of any of claims 1-4 wherein the ethylenically unsaturated organic compound is acrylic acid, methacrylic acid, methyl acrylate, ethyl acrylate, butyl acrylate, 2-ethylhexyl acrylate or a mixture of any thereof.

6. The method of any of claims 1-4 wherein the product or product mixture is an acrylate ester, mixture of acrylate esters, or a mixture of an acrylate ester or acrylate esters with acrylic acid.

7. The method of any of claims 1-6 wherein the distillation is carried out under vacuum at pressures less than 500 mm Hg absolute at temperatures in the range of 50°C to 170°C.

8. A composition of a polymerizable ethylenically unsaturated organic compound inhibited from unintentional or premature polymerization in the liquid phase by the addition of an inhibiting amount of a liquid phase inhibitor system containing a phenolic inhibitor and a soluble manganese or cerium alkanoate of a saturated $C_5$ to $C_{19}$ monocarboxylic acid.

9. The composition of claim 8 wherein the cerium or manganese alkanoate (expressed as ppm of the manganese or cerium metal) is present in an amount ranging from 1 part per million to 5,000 parts per million and the phenolic inhibitor is present in an amount ranging from 5 parts per million to 1000 parts per million.

10. The composition of claim 9 wherein the cerium or manganese alkanoate (expressed as ppm of the manganese or cerium metal) is present in an amount ranging from 1 part per million to 500 parts per million and the phenolic type inhibitor is present in an amount ranging from 25 parts per million to 500 parts per million.

11. The composition of any of claims 8-10 wherein the phenolic inhibitor is selected from hydroquinone, the monomethyl ether of hydroquinone, catechol, guaiacol, and resorcinol.

12. The composition of any of claims 8-11 wherein the soluble manganese or cerium compound is manganese heptanoate or manganese pelargonate.

13. The composition of any of claims 8-12 wherein the polymerizable ethylenically unsaturated organic compound is acrylic acid, methacrylic acid; methyl acrylate, ethyl acrylate, butyl acrylate, 2-ethylhexyl acrylate or a mixture of any thereof.